# EUROPÄISCHE PATENTANMELDUNG

(11) **EP 1 084 725 A1**
(43) Veröffentlichungstag der Anmeldung: **21.03.2001**
(21) Anmeldenummer: 00250305.0
(22) Anmeldetag: 14.09.2000
(51) Int. Cl.: A61M 5/44

(54) **Vorrichtung zum Erwärmen eines Fluids vor dem Einleiten in einen menschlichen oder tierischen Körper mittels Kristallisation einer unterkühlten Lösung**

(30) Priorität: 17.09.1999 DE 19944764
(71) Anmelder: W.O.M. World of Medicine GmbH, 10553 Berlin (DE)
(72) Erfinder: Lutz Andreas, 12623 Berlin (DE); Wiest, Peter P., 10553 Berlin (DE)
(74) Vertreter: Lüke, Dierck-Wilm

(57) **Zusammenfassung**

Die Erfindung betrifft eine Vorrichtung zur Einleitung eines Fluids in einen menschlichen oder tierischen Körper mit einer Fluidgasquelle 2, mit einem ärztlichen Instrument 8 mit Fluidauslaßöffnung 9, mit einer Rohr- und/oder Schlauchverbindung 4,6 zwischen der Fluidquelle 2 und einer Fluideinlaßöffnung 7 des ärztlichen Instruments 8 und mit einer Einrichtung zur Fluiderwärmung 5. Die Erfindung ist dadurch gekennzeichnet, daß als Einrichtung zur Fluiderwärmung 5 eine unterkühlte Flüssigkeit 10 verwendet wird, wobei die unterkühlte Flüssigkeit 10 in thermischen Kontakt mit dem Fluid steht, jedoch von dem Fluid getrennt ist und wobei die unterkühlte Flüssigkeit 10 mittels eines Kristallisationselements 11 zur Kristallisation anregbar ist.

## Beschreibung

### Gebiet der Erfindung

Die Erfindung betrifft eine Vorrichtung zur Einleitung eines Fluids in einen menschlichen oder tierischen Körper mit einer Fluidquelle, mit einem ärztlichen Instrument mit Fluidauslaßöffnung, mit einer Rohr- und/oder Schlauchverbindung zwischen der Fluidquelle und einer Fluideinlaßöffnung des ärztlichen Instruments und mit einer Einrichtung zur Fluiderwärmung. Der Begriff der Fluide umfaßt alle fließfähigen Stoffe. Hierunter fallen insbesondere Gase, Flüssigkeiten und Suspensionen. Im Bereich der Medizin ergeben sich in vielfältigen Zusammenhängen Notwendigkeiten zur Einleitung eines Fluids in einem menschlichen oder tierischen Körper. Die Einleitung von Gas kann beispielsweise im Zusammenhang mit endoskopischen Eingriffen bei der Laparoskopie zur Aufdehnung von Körperhöhlen erforderlich sein. Flüssigkeiten können als Infusions- oder Transfusionsflüssigkeiten eingeleitet werden. Als ärztliches Instrument sind Instrumente bezeichnet, welche in den menschlichen oder tierischen Körper eingeführt werden zwecks Einleitung des Fluids. Im einfachsten Falle ist das ärztliche Instrument eine Infusionsnadel. Als ärztliche Instrumente werden weiterhin beispielhaft genannt: Endoskope, Trokare und/oder Verness-Nadeln. Eine Vorrichtung zur Einleitung eines Fluides weist in aller Regel neben den eingangs genannten Elementen auch meist elektronisch arbeitende Vorrichtungen auf, mit welchen Volumen- und/oder Massenströme sowie Drucke des Fluids gemessen und gesteuert bzw. geregelt werden können.

Im Falle der Einleitung eines Gases, beispielsweise CO₂ Gas, in eine Körperhöhle dient die Erwärmung des Gases der Verhinderung einer möglichen Unterkühlung des Patienten. Eine solche Unterkühlung droht insbesondere dann, wenn zum Ausgleich hoher Leckagen viel Gas in die Körperhöhle geleitet werden muß. Im Falle der Einleitung von Infusions- und/oder Transfusionsflüssigkeiten droht ebenfalls eine Unterkühlung des Patienten, zumal Flüssigkeiten eine vergleichsweise hohe Wärmekapazität besitzen. Mit der Erwärmung der Infusions- und/oder Transfusionsflüssigkeiten wird auch eine solche Unterkühlung vermieden.

### Stand der Technik

Vorrichtungen des eingangs genannten Aufbaues sind in diversen Ausführungen bekannt.

Im Zusammenhang mit der Gaserwärmung ist es aus der Praxis bekannt, den Schlauch mit einem Heizwiderstandsdraht sowie einem Temperaturfühler zur geregelten elektrischen Heizung des den Schlauch durchströmenden Gases einzusetzen. Aus den Literaturstellen DE-A 4319630 und DE-G 9421816 ist es bekannt, eine gasdurchflossene Kammer einzurichten, welche direkt vor dem ärztlichen Instrument angeordnet und mit einem Heizwiderstandsdraht und Temperaturfühler zur geregelten elektrischen Heizung des Gases ausgestattet ist.

Aus der Literaturstelle DE-A 2515899 ist es zur Erwärmung von Infusions- und Transfusionsflüssigkeiten, welche in Behältern bevorratet sind, bekannt, in einer um den Behälter gelegten Manschette eine exotherme chemische Reaktion ablaufen zu lassen.

Bei dem vorstehenden auf die Erwärmung von Gas bezogenen Stand der Technik ist es nachteilig, daß auf aufwendige Weise elektrische Einrichtungen und Sensoren einschließlich einer Steuerungs- und Regelungselektronik eingerichtet sein müssen. Zudem ist eine eigene elektrische Stromversorgung erforderlich. Weiterhin nachteilig ist, daß geeignete Sicherungsmaßnahmen eingerichtet werden müssen, welche bei Ausfall der Sensorik oder der Elektronik eine Überhitzung des Gases verhindern. Die Einleitung zu heißer Gase in eine Körperhöhle hätte nämlich beachtliche gesundheitliche Beeinträchtigungen zur Folge. Bei dem vorstehenden im Zusammenhang mit Infusions- und Transfusionsflüssigkeiten genannten Stand der Technik entfällt zwar der Aufwand elektrischer Einrichtungen, hierbei ist jedoch nachteilig, daß eine exotherme chemische Reaktion sich nur schwer steuern bzw. regeln läßt. Insbesondere bei geringen Volumenströmen des Fluids besteht daher die Gefahr einer zu starken Erwärmung der Flüssigkeit wiederum mit der Folge beachtlicher gesundheitlicher Beeinträchtigungen. Daher wird bei diesem Stand der Technik allein aus Sicherheitsgründen zumindest eine Überwachungseinrichtung erforderlich sein.

### Aufgabe der Erfindung

Gegenüber dem vorstehenden genannten Stand der Technik liegt der Erfindung das technische Problem zugrunde, eine Vorrichtung zur Einleitung eines Fluids in einen menschlichen oder tierischen Körper anzugeben, bei welcher das einzuleitende Fluid auf einfache Weise erwärmt wird und bei welcher eine zu starke Erwärmung des Fluids ohne aufwendige Maßnahmen sicher verhindert werden kann.

### Zusammenfassung der Erfindung

Zur Lösung dieses technischen Problems lehrt die Erfindung eine Vorrichtung, welche dadurch gekennzeichnet ist, daß als Einrichtung zur Fluiderwärmung eine unterkühlte Flüssigkeit verwendet wird, wobei die unterkühlte Flüssigkeit in thermischem Kontakt mit dem Fluid steht, jedoch von dem Fluid getrennt ist und wobei die unterkühlte Flüssigkeit mittels eines Kristallisationselements zur Kristallisation anregbar ist. Im einzelnen ist die Zusammensetzung der unterkühlten Flüssigkeit zweckmäßigerweise mit der Maßgabe gewählt, daß die Verflüssigungstemperatur nicht mehr als 80°C, vorzugsweise nicht mehr als 57°C beträgt. Es versteht sich, daß im Rahmen der Erfindung die genannten Bauelemente einfach (Zahlwort) oder mehrfach ausgebildet sein können, i.e. mit mehreren Fluidquellen, mehreren ärztlichen Instrumenten, mehreren Fluidauslaßöffnungen eines ärztlichen Instruments, mehreren Rohr- und/oder Schlauchverbindungen, mehreren Fluideinlaßöffnungen eines ärztlichen Instruments mehreren Einrichtungen zur Fluiderwärmung und/oder mehreren Kristallisationselementen gearbeitet werden kann. Entsprechendes gilt für folgend angesprochene weitere Bauelemente.

Weiterhin lehrt die Erfindung ein Verfahren zur Erwärmung eines einem menschlichen oder tierischen Körper zuzuführenden Fluids, welches dadurch gekennzeichnet ist, daß das Fluid mittels einer vom Fluid getrennten,aber mit dem Fluid in thermischem Kontakt stehenden unterkühlten Flüssigkeit durch Anregung zur Kristallisation auf eine Temperatur in dem Bereich von 25°C, vorzugsweise 33°C, bis 37°C erwärmt wird. In Ausnahmefällen, beispielsweise bei beachtlichen Wärmeverlusten im weiteren Verlauf von Fluidleitungen oder besonderen medizinschen Bedingungen können auch Temperaturen bis zu 45°C, vorzugsweise bis zu 40°C, eingerichtet sein, wobei stets darauf zu achten ist, daß das Fluid im Bereich der Fluidauslaßöffnung des ärztlichen Instruments nicht störend wärmer als die Körpertemperatur eines Patienten ist.

Schließlich lehrt die Erfindung ein Wärmelement zur Durchführung des erfindungsgemäßen Verfahrens, welches dadurch gekennzeichnet ist, daß es die folgenden Bauelemente aufweist: a) ein Behältnis enthaltend unterkühlte Flüssigkeit, b1) eine in der unterkühlten Flüssigkeit angeordnete biegbare gekerbte Blechfahne und/oder b2) einen von der unterkühlten Flüssigkeit getrennten und mittels einer Freigabevorrichtung mit dieser in Kontakt bringbaren Keimkristall, und c1) eine durch das Behältnis verlaufende rohrförmige Durchführung, wodurch das Fluid direkt leitbar oder worin die Rohr- und/oder Schlauchverbindung angeordnet ist, und/oder c2) einen einen Mantelraum bildenden Mantel, wobei das Fluid durch den Mantelraum leitbar ist.

Eine unterkühlte Flüssigkeit ist eine Flüssigkeit, welche sich bei der Temperatur, auf welcher sie sich befindet, entgegen den thermodynamischen Gleichgewichtseigenschaften in der flüssigen Phase und nicht in der festen Phase befindet. Der Begriff der unterkühlten Flüssigkeit ist hierbei im weitesten Sinne zu verstehen. Im einfachsten Fall handelt es sich um eine Flüssigkeit aus einer oder aus mehreren Komponenten, deren bei geeigneter Temperaturerniedrigung entstehende feste Phase homogen ist. Bei Mehrkomponentensystemen kann es sich allerdings bei der festen Phase auch um ein Eutektikum handeln, i.e. eine Mischung aus mehreren festen Phasen, gegebenenfalls in Anwesenheit einer flüssigen Phase. Eine unterkühlte Flüssigkeit im Rahmen der Erfindung ist aber insbesondere eine wässrige Salzlösung, wobei das Löslichkeitsprodukt bei der Temperatur der Unterkühlung überschritten ist. Allen vorstehenden Fällen ist gemeinsam, daß der entgegen der thermodynamischen Gleichgewichtsdaten bei vergleichsweise niedrigen Temperaturen erhältliche flüssige Aggregatzustand das ergebnis einer kinetischen Hemmung ist. Diese kinetische Hemmung läßt sich dadurch überwinden, daß auf geeignete Weise der Solidifierungsprozeß initiiert wird. Sobald der Solidifierungsprozeß initiiert ist, läuft das Sytem in den thermodynamischen Gleichgewichtszustand ein, i.e. es tritt die thermodynamisch zu erwartende Phasenumwandlung bzw. Phasenverteilung ein. Im Falle der Salzlösung ist dieser Gleichgewichtszustand durch das Löslichkeitsprodukt bei der Umgebungstemperatur gegeben; die darüber hinausgehende Menge an Salz kristallisiert unter Freisetzung der Lösungsenthalpie aus. Allen vorstehenden Fällen ist gemeinsam, daß bei der (ggf. Teil-) Solidifizierung Wärme frei wird.

Die Erfindung beruht zunächst auf der Erkenntnis, daß der Einsatz einer unterkühlten Flüssigkeit eine inhärente Sicherheit gegen übermäßige Wärmeabgabe besitzt. Wenn nämlich die unterkühlte Flüssigkeit in Verfolg des Solidifizierungsprosses und der damit verbundenen Wärmeabgabe eine Temperatur erreicht, bei welcher aufgrund der thermodynamischen Eigenschaften eine Solidifizierung nicht mehr stattfindet, so stoppt dieser Prozeß automatisch. So kann durch Auswahl der Komponenten der unterkühlten Flüssigkeit entsprechend der thermodynamischen Daten eine Maximaltemperatur und damit auch eine Maximaltemperatur des zu erwärmenden Fluids praktisch beliebig eingestellt werden. Das System muß sich ansonsten lediglich zur Unterkühlung eignen. Die Erfindung beruht hieran anschließend auf der weiteren Erkenntnis, daß insbesondere bei strömenden Fluiden somit eine Überhitzung des Fluides ausgeschlossen wird, und zwar völlig unabhängig davon, wie hoch oder niedrig der Volumenstrom des Fluides durch die Einrichtung zur Fluiderwärmung ist.

Es versteht sich, daß die unterkühlte Flüssigkeit von dem Fluid durch Wandungen geeigneten Materials abgetrennt ist. Geeignete Materialien sind bezüglich der unterkühlten Flüssigkeit diffusionsdicht, hinreichend temperaturbeständig und nicht geeignet zur Auslösung der Kristallisation. Letzteres erfordert, daß die mit der unterkühlten Flüssigkeit in Kontakt befindliche Wandung eine hinreichend niedrige Oberflächenrauhheit aufweist, bei Verformung, insbesondere Biegung, nicht Risse, insbesondere keine oberflächennahe Mikrorisse bildet und auch in chemischer Hinsicht nichts zu einer Kristallisation beiträgt. Der grundsätzliche Aufbau einer solchen Einrichtung zur Fluiderwärmung entspricht einem Wärmetauscherelement.

Mit der Erfindung werden die folgenden Vorteile erreicht. Zunächst wird aufgrund der inhärenten thermodynamischen Eigenschaften der unterkühlten Flüssigkeit eine Übertemperatur des Fluids sicher und ohne jegliche Zusatzmaßnahmen verhindert. Im Zusammenhang hiermit steht, daß aufwendige und störanfällige elektrische Einrichtungen zur Heizung und eine dazugehörige Sensorik mit Steuer- und/oder Regelelementen entbehrlich sind. Ein weiterer Vorteil ist, daß die Einrichtung zur Fluiderwärmung nach der Kristallisation nicht notwendigerweise verworfen werden muß, sondern durch Erwärmen über die Kristallisationstemperatur hinaus wieder verflüssigt und dann unterkühlt werden kann. Insofern ist die Einrichtung zur Fluiderwärmung regenerierbar, wobei die Zahl der Regenerierungszyklen praktisch unbeschränkt ist. Demgegenüber ist eine exothermische chemische Reaktion nicht oder nur mit großem Aufwand reversibel. Selbstverständlich ist es auch möglich, die Einrichtung zur Fluiderwärmung im Rahmen der Erfindung als preiswerten Einmal-Artikel herzustellen.

### Detaillierte Beschreibung bevorzugter Ausführungsformen

Besonders vorteilhaft ist es im Rahmen der Erfindung, wenn das Fluid ein Gas, vorzugsweise CO₂ Gas, ist. Die Einrichtung zur Fluiderwärmung läßt sich dann aufgrund der bei der Kristallisation entstehenden Wärmemengen in Verbindung mit der geringen Wärmekapazität von Gas besonders einfach und wenig voluminös aufbauen.

Bevorzugt ist es, daß die unterkühlte Flüssigkeit eine hochkonzentrierte wässrige Salzlösung, vorzugsweise eine Natriumacetatlösung, ist. Die Kristallisationstemperatur des Natriumacetats läßt sich hierbei in weiten Grenzen durch Einstellung des Mengenverhältnisses Natriumacetat/Wasser vorgeben. Dieses Mengenverhältnis wird so gewählt, daß die Natriumacetatlösung bis wenigstens auf die Umgebungstemperatur, bei welcher die Einrichtung zur Fluiderwärmung eingesetzt werden soll, unterkühlt werden kann.

Außerdem sollte der Anteil an Natriumacetat nicht so groß sein, daß die Kristallisation beispielsweise durch Schütteln u.dgl. unbeabsichtigt ausgelöst wird, wenn sie sich auf der Umgebungs- bzw. Verwendungstemperatur befindet. Andererseits muß der Anteil an Natriumacetat ausreichend groß sein, um eine sichere Anregung der Kristallisation durch das Kristallisationselement zu gewährleisten. Bevorzugte Mengenverhältnisse Natriumacetat/Wasser liegen in dem Bereich von 100g Natriumacetat zu 50g Wasser bis 100g Natriumacetat zu 100g Wasser. Hiermit lassen sich bei ausreichender Stabilität der unterkühlten Flüssigkeit (unterkühlbar bis zu 20°C, 15°C, oder bis zu 5°C und tiefer) bei Raumtemperatur (18°C bis 22°C, insbesondere ca. 20°C) Kristallisationstemperaturen in dem Bereich von 57°C (100g/50g) bis 37°C (100g/100g) einstellen. Die Kristallisationstemperatur beschreibt die Temperatur, bei welcher im thermodynamischen Gleichgewicht die angegebene Menge an Natriumacetat in der angegebenen Menge an Wasser lösbar ist (gesättigte Lösung). Andere (Gleichgewichts-) Werte lassen sich unschwer aus den thermodynamischen Daten der verwendeten Stoffe errechnen.

Die Verwendung von Natriumacetatlösungen im Bereich der Gesundheitspflege ist an sich aus der Literaturstelle DE-C 2734673 bekannt. Im Rahmen der insofern bekannten Maßnahmen befindet sich die Natriumacetatlösung in einem dicht verschlossenen flexiblen Behälter, in welchem ein Kristallisationselement zur Kristallisation angebracht ist, und welcher nach Initiierung der Kristallisation auf ein Körperteil aufgelegt wird zwecks Erwärmung desselben. In diesem Zusammehang stellt sich jedoch die Problematik der sicheren und zuverlässigen Erwärmung von strömenden Fluiden nicht.

In baulicher Hinsicht kann die Einrichtung zur Fluiderwärmung ein Wärmetauscher beliebiger Bauart sein. Es ist möglich, daß die unterkühlte Flüssigkeit in einem Behältnis mit rohrförmiger Durchführung angeordnet ist, wobei das Fluid direkt durch die rohrförmige Durchführung leitbar oder die Rohr- und/oder Schlauchverbindung in der rohrförmigen Durchführung angeordnet ist. Bei der erstgenannten Alternative versteht es sich, daß geeignete Anschlüsse beidseitig der rohrförmigen Durchführung zum Anschluß der Rohr- oder Schlauchverbindung vorgesehen sind. Bei der zweitgenannten Alternative kann die Schlauchverbindung durch die rohrförmige Durchführung hindurch schiebbar sein, es ist aber auch möglich, das Behältnis so zu gestalten, daß die Schlauchverbindung in die rohrförmige Durchführung eingelegt wird. Bespielsweise kann das Behältnis zumindest in dem Bereich der rohrförmigen Durchführung teilbar ausgebildet sein. Es ist aber auch möglich, die rohrförmige Durchführung wandständig vorzusehen, wobei dann nur ein Teil der Mantelfläche der rohrförmigen Durchführung erwärmt wird. Ein besonders hoher Wärmeübertrag wird erreicht, wenn die rohrförmige Durchführung im Inneren des Behältnisses spiralförmig verläuft. Hierdurch läßt sich durch Einrichtung einer relativ großen Querschnittsfläche für den Wärmestrom ein vergleichsweiser hoher Wärmefluß einstellen. Selbstverständlich sind auch andere Maßnahmen zur Erhöhung der wärmeübertragenden Flächen denkbar, wie beispielsweise kühlerrippenartig ausgebildete und in die rohrförmige Durchführung ragende Elemente.

Das Kristallisationselement kann eine in der unterkühlten Flüssigkeit angeordnete biegbare gekerbte Blechfahne sein.

Durch die Biegung entstehen vermutlich im Bereich der Kerbung mikroskopische oberflächenaktive Bereiche, welche die Kristallisation initiieren. Alternativ ist es möglich, daß das Kristallisationselement ein von der unterkühlten Flüssigkeit getrennter und mittels einer Freigabevorrichtung mit dieser in Kontakt bringbarer Keimkristall, vorzugsweise Natriumacetatkristall, ist. Dabei ist die Freigabevorrichtung beliebig ausgestaltbar und muß lediglich bewirken, daß eine zwischen der unterkühlten Flüssigkeit und dem Keimkristall befindliche Wandung durchbrochen werden kann.

Im Rahmen des grundsätzlichen wärmetauscherartigen Aufbaus ist es auch möglich, daß die unterkühlte Flüssigkeit in einem Behältnis angeordnet ist, welches einen einen Mantelraum bildenden Mantel aufweist, wobei das Fluid durch den Mantelraum hindurch leitbar ist.

Ist die Einrichtung zur Fluiderwärmung zur mehrfachen Verwendung vorgesehen, so sollte auch das Kristallisationselement mehrfach einsetzbar sein oder es sollten eine Mehrzahl von Kristallisationselemente, welche jeweils für sich und unabhängig voneinander aktivierbar sind, eingerichtet sein. Ein Beispiel für ein mehrfach einsetzbares Kristallisationselement ist die vorstehend angesprochene biegbare gekerbte Blechfahne. Dann kann die Anzahl der Verwendungszyklen durch die Anzahl der Kerben vorgegeben werden. Es ist aber auch nicht ausgeschlossen, daß eine Kristallisation mehrfach auslösbar ist durch jeweiliges Hin- und Herbiegen im Bereich einer einzigen Kerbe. Eine Mehrzahl von Kristallisationselementen kann beispielsweise durch in als Ampullen ausgebildeten Freigabevorrichtungen eingeschlossene Keimkristalle eingerichtet sein. Mit dem Bruch einer

Ampulle wird ein oder mehrere Keimkristalle in die unterkühlte Flüssigkeit freigesetzt mit der Folge der Kristallisation. Bei jedem Verwendungszyklus wird folglich eine Freigabevorrichtung verbraucht. Die Ampullentechnologie bzw. entsprechende Technologien können selbsterständlich auch im Rahmen von Einwegeinrichtungen zur Fluiderwärmung eingesetzt sein; dann wird in der Regel lediglich eine Ampulle vorgesehen sein, ggf. ergänzt durch Reserveampullen.

### Ausführungsbeispiele

Im Folgenden wird die Erfindung anhand von lediglich Ausführungsbeispiele darstellenden Figuren näher erläutert. Es zeigen:
- Fig. 1:: Eine Gesamtansicht einer erfindungsgemäßen Vorrichtung,
- Fig. 2:: eine erste Ausführungsform einer Einrichtung zur Fluiderwärmung,
- Fig. 3:: eine zweite Ausführungsform einer Vorrichtung zur Fluiderwärmung und
- Fig. 4:: eine dritte Ausführungsform einer Vorrichtung zur Fluiderwärmung.

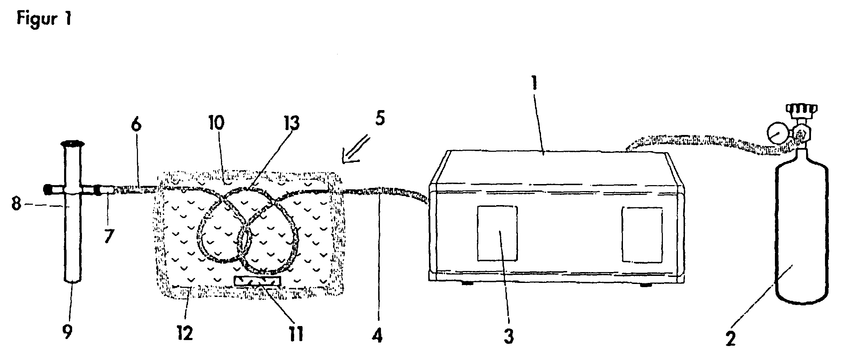

In der Fig. 1 erkennt man eine Vorrichtung zur Einleitung eines Fluids in einem menschlichen oder tierischen Körper mit einem Hauptgerät 1. Das Hauptgerät 1 umfaßt eine Fluidquelle 2, im Ausführungsbeispiel eine CO₂-Quelle, und Steuerungs- und Regelungseinheiten 3 für Gasdruck und Gasfluß. Hieran angeschlossen ist eine erste Schlauchverbindung 4, deren anderes Ende an einer Einrichtung zur Fluiderwärmung 5 angeschlossen ist. Hiervon führt eine zweite Schlauchverbindung 6 zu einer Fluideinlaßöffnung 7 eines ärztlichen Instrumentes 8, welches eine Fluidauslaßöffnung 9 aufweist. Im Betrieb wird der Bereich des ärztlichen Instruments 8 mit der Fluidauslaßöffnung 9 in den menschlichen oder tierischen Körper eingeführt und so die Einleitung des Gases durchgeführt. Die Einrichtung zur Fluiderwärmung 5 kann auch direkt an oder in dem Hauptgerät 1 oder dem ärztlichen Instrument 8 vorgesehen sein.

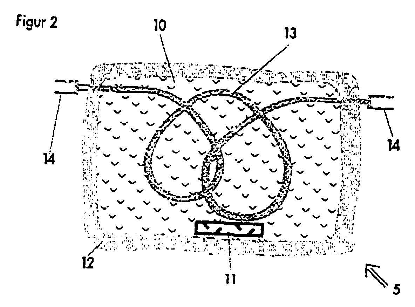

Den Ausführungsbeispielen der Figuren 2 bis 4 ist gemeinsam, daß die Einrichtung zur Fluiderwärmung 5 eine unterkühlte Flüssigkeit 10 enthält, welche mit dem sie durchströmenden Gas in thermischem Kontakt steht. Die unterkühlte Flüssigkeit 10 ist jedoch von dem Gas getrennt und kann dieses nicht kontaminieren. In allen Ausführungsbeispielen ist ein Kristallisationselement 11 eingerichtet, mittels welchem die Kristallisation der unterkühlten Flüssigkeit anregbar ist. In allen dargestellten Ausführungsbeispielen ist die unterkühlte Flüssigkeit 10 eine Lösung aus Natriumacetat in Wasser, wobei das Gewichtsverhältnis Natriumacetat zu Wasser 100g/50g beträgt. Diese Flüssigkeit läßt sich stabil auf eine Temperatur von bis zu 15°C und tiefer °C unterkühlen und die Kristallisationstemperatur beträgt 57°C.

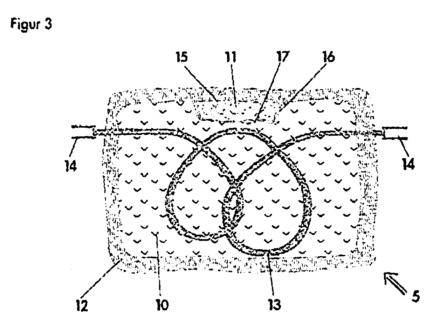

In dem Ausführungsbeispiel der Figuren 2 und 3 ist die unterkühlte Flüssigkeit 10 in einem flexiblen Behältnis 12 angeordnet. Das Behältnis 12 ist aus einer PVC-Folie gebildet. Im Inneren des Behältnisses 12 ist eine spiralförmige rohrförmige Durchführung 13 angeordnet. Das Gas ist direkt durch die rohrförmige Durchführung 13 leitbar. Hierzu besitzt die rohrförmige Durchführung 13 an ihren jeweiligen Enden und außenseitig an dem Behältnis 12 befestigt Schlauchanschlußelemente 14.

In dem Ausführungsbeispiel der Fig. 2 ist das Kristallisationselement 11 eine in der unterkühlten Flüssigkeit 10 angeordnete, biegbare gekerbte Blechfahne.

In dem Ausführungsbeispiel der Fig. 3 ist das Kristallisationselement 11 ein von der unterkühlten Flüssigkeit 10 getrennter und mittels einer Freigabevorrichtung 15 mit dieser in Kontakt bringbarer Keimkristall, nämlich ein Natriumacetatkristall. Die Freigabevorrichtung trennt den Natriumacetatkristall von der unterkühlten Flüssigkeit 10. Im Rahmen der Freigabevorrichtung 15 ist eine starre Wandung 16 mit einer Sollbruchstelle 17 eingerichtet. Zur Anregung der Kristallisation in der unterkühlten Flüssigkeit 10 wird manuell ein Bruch der Sollbruchstelle 17 bewirkt mit der Folge, daß die unterkühlte Flüssigkeit 10 mit dem Keimkristall in Kontakt kommt und die Kristallisation initiiert wird.

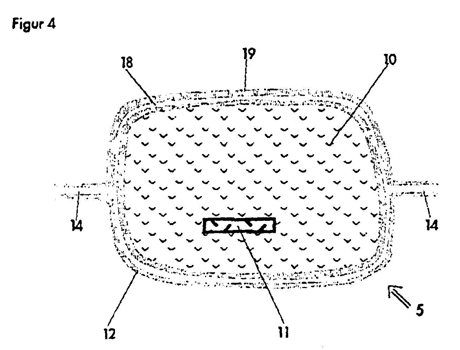

In dem Ausführungsbeispiel der Fig. 4 ist die unterkühlte Flüssigkeit 10 in einem Behältnis 12 angeordnet, welches einen einen Mantelraum 18 bildenden Mantel 19 aufweist. Durch den Mantelraum 18 ist das Gas hindurchleitbar, wie der Fig. 4 ohne weiteres entnehmbar.

## Patentansprüche

1. Vorrichtung zur Einleitung eines Fluids in einen menschlichen oder tierischen Körper mit einer Fluidquelle (2), mit einem ärztlichen Instrument (8) mit Fluidauslaßöffnung (9), mit einer Rohr- und/oder Schlauchverbindung (4,6) zwischen der Fluidquelle (2) und einer Fluideinlaßöffnung (7) des ärztlichen Instruments (8) und mit einer Einrichtung zur Fluiderwärmung (5),
dadurch gekennzeichnet,
daß als Einrichtung zur Fluiderwärmung (5) eine unterkühlte Flüssigkeit (10) verwendet wird, wobei die unterkühlte Flüssigkeit (10) in thermischem Kontakt mit dem Fluid steht, jedoch von dem Fluid getrennt ist und wobei die unterkühlte Flüssigkeit (10) mittels eines Kristallisationselements (11) zur Kristallisation anregbar ist.

2. Vorrichtung nach Anspruch 1, dadurch gekennzeichnet, daß das Fluid ein Gas, vorzugsweise CO₂ Gas, ist.

3. Vorrichtung nach Anspruch 1 oder 2, dadurch gekennzeichnet, daß die unterkühlte Flüssigkeit (10) eine hochkonzentrierte wässrige Salzlösung, vorzugsweise eine Natriumacetatlösung ist.

4. Vorrichtung nach einem der Ansprüche 1 bis 3, dadurch gekennzeichnet, daß die unterkühlte Flüssigkeit (10) in einem Behältnis (12) mit rohrförmiger Durchführung (13) angeordnet ist und daß das Fluid direkt durch die rohrförmige Durchführung (13) durchleitbar oder die Rohr- und/oder Schlauchverbindung (4,6) in der rohrförmigen Durchführung (13) angeordnet ist.

5. Vorrichtung nach einem der Ansprüche 1 bis 4, dadurch gekennzeichnet, daß die rohrförmige Durchführung (13) im Inneren des Behältnisses (12) spiralförmig verläuft.

6. Vorrichtung nach einem der Ansprüche 1 bis 3, dadurch gekennzeichnet, daß die unterkühlte Flüssigkeit (10) in einem Behältnis (12) angeordnet ist, welches einen einen Mantelraum (18) bildenden Mantel (19) aufweist, wobei das Fluid durch den Mantelraum (18) hindurch leitbar ist.

7. Vorrichtung nach einem der Ansprüche 1 bis 6, dadurch gekennzeichnet, daß das Kristallisationselement (11) eine in der unterkühlten Flüssigkeit (10) angeordnete biegbare gekerbte Blechfahne oder ein von der unterkühlten Flüssigkeit (10) getrennter und mittels einer Freigabevorrichtung (15) mit dieser in Kontakt bringbarer Keimkristall, vorzugsweise Natriumacetatkristall, ist.

8. Verfahren zur Erwärmung eines einem menschlichen oder tierischen Körper zuzuführenden Fluids, dadurch gekennzeichnet, daß das Fluid mittels einer vom Fluid getrennten aber mit dem Fluid in thermischen Kontakt stehender unterkühlten Flüssigkeit durch Anregung zur Kristallisation auf eine Temperatur in dem Bereich von 33°C bis 37°C erwärmt wird.

9. Verfahren nach Anspruch 8, dadurch gekennzeichnet, daß eine Vorrichtung nach einem der Ansprüche 1 bis 7 zur Erwärmung verwendet wird.

10. Wärmelement zur Durchführung eines Verfahrens nach Anspruch 8 oder 9, dadurch gekennzeichnet, daß es die folgenden Bauelemente aufweist:
a) ein Behältnis (12) enthaltend unterkühlte Flüssigkeit (10)
b1) eine in der unterkühlten Flüssigkeit (10) angeordnete biegbare gekerbte Blechfahne und/oder
b2) einen von der unterkühlten Flüssigkeit (10) getrennten und mittels einer Freigabevorrichtung (15) mit dieser in Kontakt bringbarer Keimkristall, und
c1) eine durch das Behältnis (12) verlaufende rohrförmige Durchführung (13), wodurch das Fluid direkt leitbar oder worin die Rohr- und/oder Schlauchverbindung (4,6) angeordnet ist, und/oder
c2) einen einen Mantelraum (18) bildenden Mantel (19), wobei das Fluid durch den Mantelraum (18) leitbar ist.
